(19) Europäisches Patentamt
European Patent Office
Office européen des brevets

(11) Veröffentlichungsnummer: **0 040 366**
**B1**

(12) # EUROPÄISCHE PATENTSCHRIFT

(45) Veröffentlichungstag der Patentschrift: **02.11.83**

(51) Int. Cl.³: **A 01 N 43/50,** C 07 D 233/60 // C07D233/61

(21) Anmeldenummer: **81103477.6**

(22) Anmeldetag: **07.05.81**

(54) **Imidazolyl-vinyl-ketone und -carbinole, Verfahren zu ihrer Herstellung sowie ihre Verwendung als Fungizide.**

(30) Priorität: **19.05.80 DE 3019044**

(43) Veröffentlichungstag der Anmeldung: **25.11.81 Patentblatt 81/47**

(45) Bekanntmachung des Hinweises auf die Patenterteilung: **02.11.83 Patentblatt 83/44**

(84) Benannte Vertragsstaaten: **AT BE CH DE FR GB IT LI NL**

(56) Entgegenhaltungen:
**DE - A - 2 738 640**
**GB - A - 2 004 276**
**US - A - 4 182 862**

(73) Patentinhaber: **BAYER AG, Zentralbereich Patente, Marken und Lizenzen, D-5090 Leverkusen 1, Bayerwerk (DE)**

(72) Erfinder: **Elbe, Hans-Ludwig, Dr., Dasnoeckel 59, D-5600 Wuppertal 11 (DE)**
Erfinder: **Büchel, Karl Heinz, Prof. Dr., Dabringhausener Strasse 42, D-5063 Burscheid (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 040 366 B1

Imidazolylvinylketone und -carbinole, Verfahren zu ihrer Herstellung sowie ihre Verwendung als Fungizide

Die vorliegende Erfindung betrifft neue Imidazolylvinylketone und -carbinole, ein Verfahren zu ihrer Herstellung sowie ihre Verwendung als Fungizide.

Es ist bereits bekannt geworden, dass bestimmte 1-Phenyl-2-imidazolyl-4,4-dimethyl-1-penten-3-one gute fungizide Wirksamkeit besitzen (vgl. DE-OS Nr. 2838847). Ausserdem ist bekannt, dass bestimmte Imidazolyläther, wie z.B. 1-(4-Chlorphenoxy)-2-(4-chlorbenzyloxy)-3,3-dimethyl-1-imidazol-1-ylbutan, fungizide Eigenschaften aufweisen (vgl. DE-OS Nr. 2720949). Die Wirkung dieser Verbindungen ist jedoch, insbesondere bei niedrigen Aufwandmengen und -konzentrationen, nicht immer ganz befriedigend. Ebenfalls bekannt sind 1-Triazolyl- und 1-Imidazolylvinylderivate mit guten herbiziden Eigenschaften (DE-OS Nr. 2738640). Weiterhin sind Verfahren zur Herstellung von fungizid wirksamen 1,3-disubstituierten 2-Azolyl-2-propen-1-onen bekannt (US-PS Nr. 4182862); auch eine vorgängige europäische Patentanmeldung (Veröffentlichungsnummer 0032239) betrifft ein Verfahren zur Herstellung von fungizid wirksamen 1-Azolyl-1-carbonylvinylverbindungen.

Es wurden neue Imidazolylvinylketone und -carbinole der allgemeinen Formel

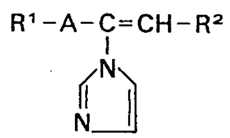
(I)

in welcher

A für die Ketogruppe oder eine CH(OH)-Gruppierung steht,

$R^1$ für tert.-Butyl, Chlor-tert.-butyl, Fluor-tert.-butyl, Dichlor-tert.-butyl und Difluor-tert.-butyl steht, und

$R^2$ für Alkyl mit 1 bis 29 Kohlenstoffatomen, für Cycloalkyl und Cycloalkenyl mit 5 bis 7 Kohlenstoffatomen und für Halogenalkyl mit 1 bis 18 Kohlenstoff- und 1 bis 5 Halogenatomen steht, weiterhin für Alkoxyalkyl und Alkylmercaptoalkyl mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatomen im Alkylteil steht, sodann für Hydroxyalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen und durch Alkyl, Alkoxy und Alkylthio mit jeweils 1 bis 4 Kohlenstoffatomen, und ausserdem für Benzyl steht, welches im Phenylteil durch die weiter oben genannten Phenylsubstituenten und die 1,1,2-Trifluor-2-chlorethoxygruppe und im Alkylteil durch Cyano substituiert sein kann, und schliesslich noch für die Indenyl-, die Fluorenyl-, die Diphenylmethyl- und die Triphenylmethylgruppe steht,

und deren physiologisch verträglichen Säureadditionssalze sowie Metallsalzkomplexe gefunden.

Die Verbindungen der Formel I können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an. Wenn A für die CH(OH)-Gruppierung steht, liegt ein asymmetrisches Kohlenstoffatom vor, so dass die Verbindungen der Formel I in diesem Fall ausserdem in zwei optischen Isomerenformen anfallen; vorzugsweise fallen sie als Racemate an. Die vorliegende Erfindung betrifft sowohl die einzelnen Isomeren als auch die Isomerenchemische.

Weiterhin wurde gefunden, dass man die Imidazolylvinylketone und -carbinole der Formel I erhält, wenn man Ketoenamine der Formel

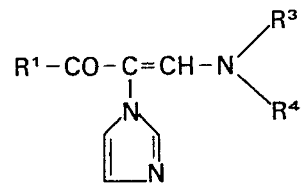
(II)

in welcher

$R^1$ die oben angegebene Bedeutung hat, und

$R^3$ und $R^4$ gleich oder verschieden sind und für Alkyl mit 1 bis 4 Kohlenstoffatomen stehen,

mit magnesium-organischen Verbindungen der Formel

$$Hal-Mg-R^2 \qquad (III)$$

in welcher

$R^2$ die oben angegebene Bedeutung hat, und

Hal für Halogen steht,

in Gegenwart eines Lösungsmittels umsetzt, und gegebenenfalls die entstehenden Ketoderivate der Formel I in üblicher Weise reduziert.

An die so erhaltenen Verbindungen der Formel I kann gegebenenfalls anschliessend eine Säure oder ein Metallsalz addiert werden.

Die neue Imidazolylvinylketone und -carbinole der Formel I weisen starke fungizide Eigenschaften auf. Dabei zeigen überraschenderweise die erfindungsgemässen Verbindungen eine bessere fungizide Wirksamkeit als die aus dem Stand der Technik bekannten 1-Phenyl-2-imidazolyl-4,4-dimethyl-1-penten-3-one, welche chemisch und wirkungsmässig naheliegende Verbindungen sind, und als das ebenfalls aus dem Stand der Technik bekannte 1-(4-Chlorphenoxy)-2-(4-chlorbenzyloxy)-3,3-dimethyl-1-imidazol-1-ylbutan, welches wirkungsmässig eine naheliegende Verbindung ist. Die erfindungsgemässen Stoffe stellen somit eine Bereicherung der Technik dar.

Die erfindungsgemässen Imidazolylvinylketone und -carbinole sind durch die Formel I definiert.

Im einzelnen seien ausser den bei den Herstellungsbeispielen genannten Verbindungen die folgenden Verbindungen der allgemeinen Formel I genannt:

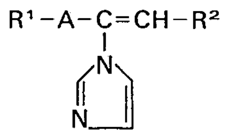 (I)

| $R^1$ | $R^2$ | A |
|---|---|---|
| $(CH_3)_3C-$ | $-CH(C_2H_5)_2$ | CO |
| $(CH_3)_3C-$ | $-C(CH_3)_3$ | CO |
| $(CH_3)_3C-$ | $-CH_2-$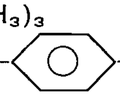$-OCF_2CHFCl$ | CO |
| $(CH_3)_3C-$ | $-CH_2-CH_2-O-C_3H_7-n$ | CO |
| $(CH_3)_3C-$ | $-C_{23}H_{47}-n$ | CO |
| $(CH_3)_3C-$ | $-CH_2-CH_2-S-C_3H_7-n$ | CO |
| $(CH_3)_3C-$ | $-(CH_2)_3-N(CH_3)_2$ | CO |
| $(CH_3)_3C-$ | $-(CH_2)_7-OH$ | CO |
| $(CH_3)_3C-$ | $-C(CH_3)=CH_2$ | CO |
| $(CH_3)_3C-$ | $-C(CH_3)=C(CH_3)_2$ | CO |
| $(CH_3)_3C-$ | $-(CH_2)_7F$ | CO |
| $(CH_3)_3C-$ | $-(CH_2)_7Cl$ | CO |
| $(CH_3)_3C-$ | 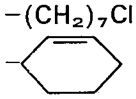 | CO |
| $(CH_3)_3C-$ | 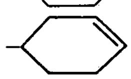 | CO |
| $ClCH_2-C(CH_3)_2-$ | $-C_2H_5$ | CO |
| $ClCH_2-C(CH_3)_2-$ | $-CH(CH_3)(C_2H_5)$ | CO |
| $ClCH_2-C(CH_3)_2-$ | $-C_4H_9-n$ | CO |
| $ClCH_2-C(CH_3)_2-$ | $-CH(CH_3)_2$ | CO |
| $ClCH_2-C(CH_3)_2-$ | $-C(CH_3)_3$ | CO |
| $ClCH_2-C(CH_3)_2-$ | 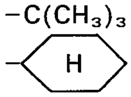 | CO |
| $ClCH_2-C(CH_3)_2-$ | $-CH_2-$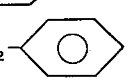 | CO |
| $ClCH_2-C(CH_3)_2-$ | $-C\equiv C-$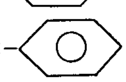 | CO |
| $FCH_2-C(CH_3)_2-$ | $-C_2H_5$ | CO |
| $FCH_2-C(CH_3)_2-$ | $-C_4H_9-n$ | CO |
| $FCH_2-C(CH_3)_2-$ | $-C(CH_3)_3$ | CO |
| $FCH_2-C(CH_3)_2-$ | $-C_7H_{15}-n$ | CO |
| $FCH_2-C(CH_3)_2-$ | 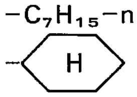 | CO |
| $FCH_2-C(CH_3)_2-$ | $-CH_2-$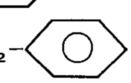 | CO |
| $FCH_2-C(CH_3)_2-$ | $-C\equiv C-$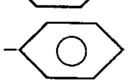 | CO |
| $CH_3-C(CH_2Cl)_2-$ | $-C_2H_5$ | CO |
| $CH_3-C(CH_2Cl)_2-$ | $-CH(CH_3)(C_2H_5)$ | CO |
| $CH_3-C(CH_2Cl)_2-$ | $-C_4H_9-n$ | CO |
| $CH_3-C(CH_2Cl)_2-$ | $-CH(CH_3)_2$ | CO |
| $CH_3-C(CH_2Cl)_2-$ | $-C(CH_3)_3$ | CO |
| $CH_3-C(CH_2Cl)_2-$ | $-C_7H_{15}-n$ | CO |
| $CH_3-C(CH_2Cl)_2-$ | 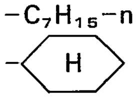 | CO |

| $R^1$ | $R^2$ | A |
|---|---|---|
| $CH_3-C(CH_2Cl)_2-$ | $-CH_2-$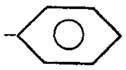 | CO |
| $CH_3-C(CH_2Cl)_2-$ | $-C\equiv C-$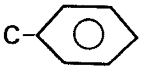 | CO |
| $CH_3-C(CH_2F)_2-$ | $-C_2H_5$ | CO |
| $CH_3-C(CH_2F)_2-$ | $-CH(CH_3)(C_2H_5)$ | CO |
| $CH_3-C(CH_2F)_2-$ | $-C_4H_9-n$ | CO |
| $CH_3-C(CH_2F)_2-$ | $-CH(CH_3)_2$ | CO |
| $CH_3-C(CH_2F)_2-$ | $-C(CH_3)_3$ | CO |
| $CH_3-C(CH_2F)_2-$ | $-C_7H_{15}-n$ | CO |
| $CH_3-C(CH_2F)_2-$ | 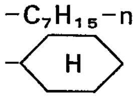 | CO |
| $CH_3-C(CH_2F)_2-$ | $-CH_2-$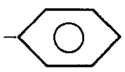 | CO |
| $CH_3-C(CH_2F)_2-$ | $-C\equiv C-$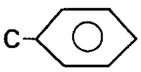 | CO |
| $(CH_3)_3C-$ | $-CH(C_2H_5)_2$ | CH(OH) |
| $(CH_3)_3C-$ | $-C(CH_3)_3$ | CH(OH) |
| $(CH_3)_3C-$ | $-CH_2-$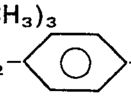$-OCF_2CHFCl$ | CH(OH) |
| $(CH_3)_3C-$ | $-CH_2-CH_2-O-C_3H_7-n$ | CH(OH) |
| $(CH_3)_3C-$ | $-C_{23}H_{47}-n$ | CH(OH) |
| $(CH_3)_3C-$ | $-CH_2CH_2-S-C_3H_7-n$ | CH(OH) |
| $(CH_3)_3C-$ | $-(CH_2)_3-N(CH_3)_2$ | CH(OH) |
| $(CH_3)_3C-$ | $-(CH_2)_7-OH$ | CH(OH) |
| $(CH_3)_3C-$ | $-C=CH_2$<br>\|<br>$CH_3$ | CH(OH) |
| $(CH_3)_3C-$ | $-C=C(CH_3)_3$<br>\|<br>$CH_3$ | CH(OH) |
| $(CH_3)_3C-$ | $-(CH_2)_7F$ | CH(OH) |
| $(CH_3)_3C-$ | $-(CH_2)_7Cl$ | CH(OH) |
| $(CH_3)_3C-$ | 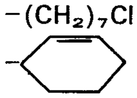 | CH(OH) |
| $(CH_3)_3C-$ | 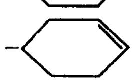 | CH(OH) |
| $ClCH_2-C(CH_3)_2-$ | $-C_2H_5$ | CH(OH) |
| $ClCH_2-C(CH_3)_2-$ | $-CH(CH_3)(C_2H_5)$ | CH(OH) |
| $ClCH_2-C(CH_3)_2-$ | $-C_4H_9-n$ | CH(OH) |
| $ClCH_2-C(CH_3)_2-$ | $-CH(CH_3)_2$ | CH(OH) |
| $ClCH_2-C(CH_3)_2-$ | $-C(CH_3)_3$ | CH(OH) |
| $ClCH_2-C(CH_3)_2-$ | 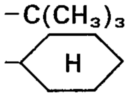 | CH(OH) |
| $ClCH_2-C(CH_3)_2-$ | $-CH_2-$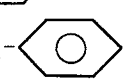 | CH(OH) |
| $ClCH_2-C(CH_3)_2-$ | $-C\equiv C-$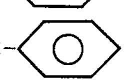 | CH(OH) |
| $FCH_2-C(CH_3)_2-$ | $-C_2H_5$ | CH(OH) |
| $FCH_2-C(CH_3)_2-$ | $-C_4H_9-n$ | CH(OH) |
| $FCH_2-C(CH_3)_2-$ | $-C(CH_3)_3$ | CH(OH) |
| $FCH_2-C(CH_3)_2-$ | $-C_7H_{15}-n$ | CH(OH) |
| $FCH_2-C(CH_3)_2-$ | 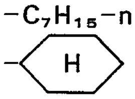 | CH(OH) |

| $R^1$ | $R^2$ | A |
|---|---|---|
| $FCH_2-C(CH_3)_2-$ | 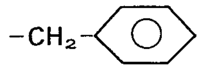 | CH(OH) |
| $FCH_2-C(CH_3)_2-$ | 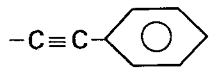 | CH(OH) |
| $CH_3-C(CH_2Cl)_2-$ | $-C_2H_5$ | CH(OH) |
| $CH_3-C(CH_2Cl)_2-$ | $-CH(CH_3)(C_2H_5)$ | CH(OH) |
| $CH_3-C(CH_2Cl)_2-$ | $-C_4H_9-n$ | CH(OH) |
| $CH_3-C(CH_2Cl)_2-$ | $-CH(CH_3)_2$ | CH(OH) |
| $CH_3-C(CH_2Cl)_2-$ | $-C(CH_3)_3$ | CH(OH) |
| $CH_3-C(CH_2Cl)_2-$ | $-C_7H_{15}-n$ | CH(OH) |
| $CH_3-C(CH_2Cl)_2-$ | 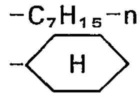 | CH(OH) |
| $CH_3-C(CH_2Cl)_2-$ | 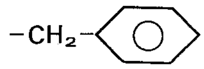 | CH(OH) |
| $CH_3-C(CH_2Cl)_2-$ | 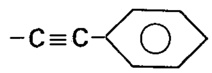 | CH(OH) |
| $(CH_3)_3C-$ | 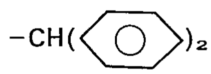 | CH(OH) |
| $(CH_3)_3C-$ | 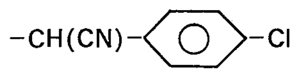 | CH(OH) |

Verwendet man beispielsweise 4,4-Dimethyl-1-dimethylamino-2-imidazol-1-yl-1-penten-3-on und tert.-Butylmagnesiumbromid als Ausgangsstoffe, so kann der Reaktionsablauf des erfindungsgemässen Verfahrens durch das folgende Formelschema wiedergegeben werden:

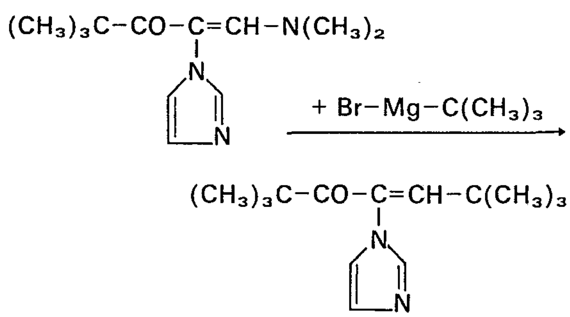


Verwendet man beispielsweise 2,2,6,6-Tetramethyl-4-imidazol-1-yl-3-hepten-5-on und Natriumborhydrid als Ausgangsstoffe, so kann der Reaktionsablauf der erfindungsgemässen Reduktion durch das folgende Formelschema wiedergegeben werden:

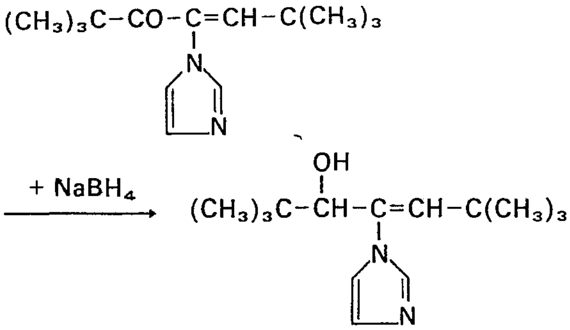


Die als Ausgangsstoffe für das erfindungsgemässe Verfahren zu verwendenden Ketoenamine sind durch die Formel II definiert. In dieser Formel stehen $R^3$ und $R^4$ insbesondere für Methyl.

Die Ketoenamine der Formel II sind Gegenstand einer eigenen älteren Patentanmeldung (vgl. die deutsche Offenlegungsschrift Nr. 3000643 und die europäischen Patentanmeldung Nr. 0032239) und können nach dem dort beschriebenen Verfahren erhalten werden, indem man Imidazolylketone der Formel

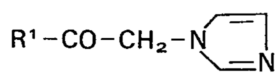 (IV)

in welcher

$R^1$ die oben angegebene Bedeutung hat,

mit Amidacetalen bzw. Aminalestern der Formeln

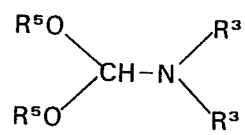 (Va)

bzw.

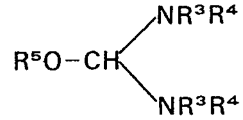 (Vb)

in welchen

$R^3$ und $R^4$ die oben angegebene Bedeutung haben, und

$R^5$ für Alkyl mit 1 bis 4 Kohlenstoffatomen steht,

in an sich bekannter Art und Weise in Gegenwart eines inerten organischen Lösungsmittels, wie

beispielsweise aromatische Kohlenwasserstoffe und wie insbesondere ein Überschuss an eingesetztem Amidacetal bzw. Aminalester der Formel Va bzw. Vb, in der Siedehitze umsetzt (vgl. hierzu auch „Chem. Ber.", *101*, 41-50 [1968]; „J. Org. Chem.", *43*, 4148-50 [1978] sowie die Herstellungsbeispiele).

Die Imidazolylketone der Formel IV sind bekannt (vgl. z.B. DE-OS Nrn. 2610022 und 2638470); bzw. lassen sie sich nach üblichen Methoden herstellen, indem man die entsprechenden Halogenketone in Gegenwart eines Säurebinders mit Imidazol umsetzt.

Die Amidacetale bzw. Aminalester der Formeln Va bzw. Vb sind allgemein bekannte Verbindungen der organischen Chemie (vgl. z.B. „Chem. Ber.", *101*, 41-50 [1968] und „J. Org. Chem.", *43*, 4248-50 [1978]).

Die ausserdem für die erfindungsgemässe Umsetzung als Ausgangsstoffe zu verwendenden magnesium-organischen Verbindungen sind durch die Formel III definiert. In dieser Formel steht Hal vorzugsweise für Chlor oder Brom.

Die magnesium-organischen Verbindungen der Formel III sind allgemein bekannte Verbindungen der organischen Chemie.

Als Lösungsmittel kommen für die erfindungsgemässe Umsetzung vorzugsweise inerte organische Lösungsmittel in reiner Form oder im Gemisch in Frage. Hierzu gehören vorzugsweise Äther, wie Diäthyläther, Methyläthyläther, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei der Durchführung des erfindungsgemässen Verfahrens in einem grösseren Bereich variiert werden. Im allgemeinen arbeitet man zwischen −50 und +150° C, vorzugsweise zwischen −20 und +120° C. Die erfindungsgemässe Umsetzung kann in Gegenwart eines Inertgases, wie Stickstoff oder Helium, durchgeführt werden. Bei der Durchführung des erfindungsgemässen Verfahrens setzt man auf 1 mol Ketoenamin der Formel II, vorzugsweise 1 bis 1,5 mol, an magnesium-organischer Verbindung der Formel III ein. Die Isolierung der Verbindungen der Formel I erfolgt in üblicher Weise.

Die erfindungsgemässe Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemässe Umsetzung polare organische Lösungsmittel in Frage. Hierzu gehören vorzugsweise Alkohole, wie Methanol, Äthanol, Butanol, Isopropanol, und Äther, wie Diäthyläther oder Tetrahydrofuran. Die Reaktion wird im allgemeinen bei 0 bis 30° C, vorzugsweise bei 0 bis 20° C, durchgeführt. Hierzu setzt man auf 1 mol des Ketons der Formel I etwa 1 mol eines komplexen Hydrids, wie Natriumborhydrid und Lithiumalanat, ein. Zur Isolierung der reduzierten Verbindungen der Formel I wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, in Frage. Die Reaktionstemperaturen können wiederum in einem grösseren Bereich variiert werden; in allgemeinen arbeitet man zwischen 20 und 120° C, vorzugsweise bei 50 bis 100° C. Zur Durchführung der Reaktion setzt man auf 1 mol des entsprechenden Ketons der Formel I etwa 1 bis 2 mol Aluminiumisopropylat ein. Zur Isolierung der reduzierten Verbindungen der Formel I wird das überschüssige Lösungsmittel durch Destillation im Vakuum entfernt und die entstandene Aluminiumverbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindung der Formel I kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure. Die Säureadditionssalze der Verbindungen der Formel I können in einfacher Weise nach üblichen Salzbildungsmethoden, z.B. durch Lösen einer Verbindung der Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der Formel I kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salzen kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe von Verbindungen der Formel I können in einfacher Weise nach üblichen Verfahren erhalten werden, so z.B. durch Lösen des Metallsalzes in Alkohol, z.B. Äthanol und Hinzufügen zur Verbindung der Formel I. Man kann Metallsalzkomplexe in bekannter Weise, z.B.

durch Abfiltrieren, Isolieren und gegebenenfalls durch Umkristallisation reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Oomyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Getreidekrankheiten, wie Gersten- bzw. Getreidemehltau *(Erysiphe graminus)* und Streifenkrankheit der Gerste, von *Venturia*-Arten, wie gegen den Erreger des Apfelschorfs *(Fusicladium dendriticum)*, von *Erysiphe*-Arten, wie gegen den Erreger des Gurkenmehltaus *(Erysiphe cichoracearum)*, sowie zur Bekämpfung der Braunfäule der Tomate *(Phytophthora infestans)* eingesetzt werden. Besonders hervorzuheben ist, dass die erfindungsgemässen Wirkstoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie halogenkohlenwasserstoffe sowie Butan, Propan, Stickstoff und Hohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylzellulose.

Es können in den Formulierungen Haftmittel wie Carboxymethylzellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert

werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g/kg Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

*Herstellungsbeispiele*

*Beispiel 1*

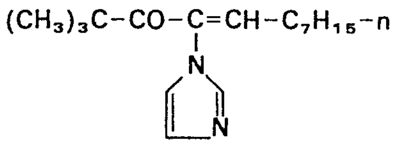

Zu 55,5 g (0,25 mol) 4,4-Dimethyl-1-dimethylamino-2-(imidazol-1-yl)-pent-1-en-3-on in 800 ml Äther gibt man bei −20° C innerhalb von 30 min unter Inertgas (Stickstoff) eine Lösung von 61 g (0,3 mol) n-Heptylmagnesiumbromid in 130 ml Äther. Nach beendeter Zugabe lässt man das Reaktionsgemisch innerhalb von ca. 2 h auf Raumtemperatur erwärmen. Danach wird mit verdünnter Salzsäure versetzt, die organische Phase wird abgetrennt, mit Wasser gewaschen, über Natriumsulfat getrocknet und eingeengt. Man erhält 54 g (78% der Theorie) 2,2-Dimethyl-4-(imidazol-1-yl)-5-dodecen-3-on vom Brechungsindex $n_D^{20}$ = 1,4802.

*Herstellung des Ausgangsproduktes*

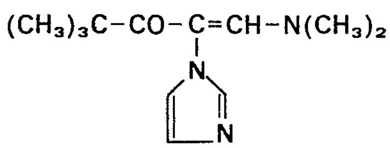

41,6 g (0,25 mol) 3,3-Dimethyl-1-(imidazol-1-yl)butan-2-on werden mit 35,7 g (0,3 mol) Dimethylformamiddimethylacetal 5 h unter Rückfluss erhitzt. Danach wird das überschüssige Acetal abdestilliert. Das zurückbleibende Öl kristallisiert beim Abkühlen. Man erhält 50 g (90,5% der Theorie) 4,4-Dimethyl-1-dimethylamino-2-(imidazol-1-yl)pent-1-en-3-on vom Schmelzpunkt 45-50° C.

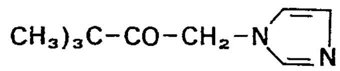

136,2 g (2 mol) Imidazol werden bei Raumtemperatur portionsweise zu 276,4 g (2 mol) gemahlenem Kaliumcarbonat und 269,2 g (2 mol) α-Chlorpinakolin in 500 ml Aceton gegeben, wobei die Innentemperatur bis zur Siedehitze ansteigt. Man lässt 5 h unter Rückfluss rühren und kühlt dann auf Raumtemperatur ab. Das Reaktionsgemisch wird filtriert und das Filtrat durch Abdestillieren des Lösungsmittels im Vakuum eingeengt. Der ölige Rückstand wird destilliert. Man erhält 296 g (89% der Theorie) 3,3-Dimethyl-1-(imidazol-1-yl)-butan-2-on vom Schmelzpunkt ~20° C.

*Beispiel 2*

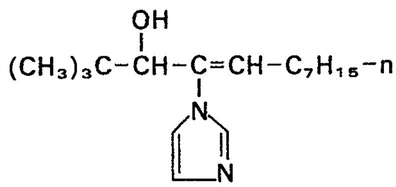

(Reduktion)

Zu 27,6 g (0,1 mol) 2,2-Dimethyl-4-(imidazol-1-yl)-5-dodecen-3-on (Beispiel 1) in 100 ml Methanol werden bei 0° C 1,1 g (0,029 mol) Natriumborhydrid, in 10 ml Wasser gelöst, getropft. Nach beendeter Zugabe lässt man 2 h bei Raumtemperatur nachrühren. Danach wird mit verdünnter Salzsäure ein pH-Wert von 6-7 eingestellt, eingeengt und der Rückstand mit Chloroform extrahiert. Die organische Phase wird über Natriumsulfat getrocknet und eingeengt. Man erhält 25,6 g (92% der Theorie) 2,2-Dimethyl-4-(imidazol-1-yl)-5-decen-3-ol vom Brechungsindex $n_D^{20}$ = 1,4860.

Analog werden die folgenden Verbindungen der allgemeinen Formel (I)

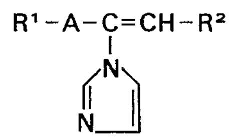 (I)

erhalten:

| Bsp. Nr. | $R^1$ | $R^2$ | A | Schmelzpunkt (°C) bzw. Brechungsindex $n_D^{20}$ |
|---|---|---|---|---|
| 3 | $(CH_3)_3C-$ | $-CH_3$ | CO | 1,5097 |
| 4 | $(CH_3)_3C-$ | $-C_2H_5$ | CO | 1,5032 |
| 5 | $(CH_3)_3C-$ | $-CH(CH_3)_2$ | CO | 1,4928 |
| 6 | $(CH_3)_3C-$ | $-C_4H_9-n$ | CO | 1,4926 |
| 7 | $(CH_3)_3C-$ | $-C(CH_3)_3$ | CO | zähfl. Öl |
| 8 | $(CH_3)_3C-$ | $-CH(CH_3)C_2H_5$ | CO | 30 |
| 9 | $(CH_3)_3C-$ | $-CH=CH_2$ | CO | zähfl. Öl |
| 10 | $(CH_3)_3C-$ | 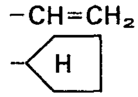 | CO | 1,5121 |

| Bsp. Nr. | $R^1$ | $R^2$ | A | Schmelzpunkt (°C) bzw. Brechungs-index $n_D^{20}$ |
|---|---|---|---|---|
| 11 | $(CH_3)_3C-$ | 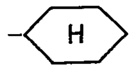 | CO | 1,5005 |
| 12 | $(CH_3)_3C-$ | $-CH_2-$ 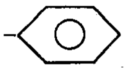 | CO | 1,5430 |
| 13 | $(CH_3)_3C-$ | $-C\equiv C-$ 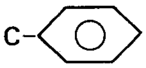 | CO | zähfl. Öl |
| 14 | $(CH_3)_3C-$ | $-CH($ 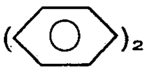 $)_2$ | CO | 35-40 |
| 15 | $(CH_3)_3C-$ | $-C_8H_{17}-n$ | CO | 1,4759 |
| 16 | $(CH_3)_3C-$ | $-C_9H_{19}-n$ | CO | 1,4769 |
| 17 | $(CH_3)_3C-$ | $-C_{10}H_{21}-n$ | CO | 1,4820 |
| 18 | $(CH_3)_3C-$ | $-C_6H_{13}-n$ | CO | 1,4835 |
| 19 | $(CH_3)_3C-$ | $-C_{12}H_{25}-n$ | CO | 1,4820 |
| 20 | $FCH_2-C(CH_3)_2-$ | $-CH(CH_3)C_2H_5$ | CO | 1,4947 |
| 21 | $FCH_2-C(CH_3)_2-$ | $-CH(CH_3)_2$ | CO | 1,5100 |
| 22 | $(CH_3)_3C-$ | 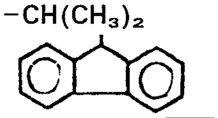 | CO | 186-187 |
| 23 | $(CH_3)_3C-$ | $-CH(CN)-$ 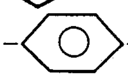 $-Cl$ | CO | 177-179 |
| 24 | $(CH_3)_3C-$ | $-CH(CH_3)_2$ | CO | 67-58 (×$CuCl_2$) |
| 25 | $(CH_3)_3C-$ | $-C_7H_{15}-n$ | CO | zähfl. Öl (×$CuCl_2$) |
| 26 | $(CH_3)_3C-$ | $-CH_3$ | CO | 76-79 (×$CuCl_2$) |
| 27 | $(CH_3)_3C-$ | $-C(CH_3)_3$ | CO | 87-90 (×$CuCl_2$) |
| 28 | $(CH_3)_3C-$ | $-CH_2-$ 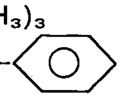 | CO | 114-120 (×$CuCl_2$) |
| 29 | $(CH_3)_3C-$ | $-CH(CH_3)C_2H_5$ | CO | 60-64 (×$CuCl_2$) |
| 30 | $(CH_3)_3C-$ | 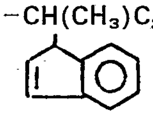 | CO | 157-161 (×$CuCl_2$) |
| 31 | $(CH_3)_3C-$ | $-C_2H_5$ | CO | 70 (×$CuCl_2$) |
| 32 | $(CH_3)_3C-$ | $-C_6H_{13}-n$ | CO | zähfl. Öl (×$CuCl_2$) |
| 33 | $(CH_3)_3C-$ | $-C_4H_9-n$ | CH(OH) | 1,4970 |
| 34 | $(CH_3)_3C-$ | $-CH_3$ | CH(OH) | 1,5106 |
| 35 | $(CH_3)_3C-$ | $-CH(CH_3)_2$ | CH(OH) | 1,4979 |
| 36 | $(CH_3)_3C-$ | $-CH(CH_3)C_2H_5$ | CH(OH) | 1,4966 |
| 37 | $(CH_3)_3C-$ | 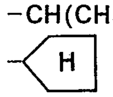 | CH(OH) | 1,5100 |
| 38 | $(CH_3)_3C-$ | $-C(CH_3)_3$ | CH(OH) | 30 |
| 39 | $(CH_3)_3C-$ | $-CH_2-$ 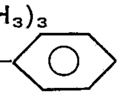 | CH(OH) | 117-125 |
| 40 | $(CH_3)_3C-$ | $-C\equiv C-$ 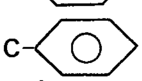 | CH(OH) | 1,5485 |
| 41 | $(CH_3)_3C-$ | 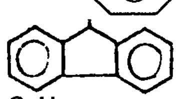 | CH(OH) | 199-201 |
| 42 | $(CH_3)_3C-$ | $-C_8H_{17}-n$ | CH(OH) | 1,4875 |
| 43 | $(CH_3)_3C-$ | $-C_9H_{19}-n$ | CH(OH) | 1,4809 |
| 44 | $(CH_3)_3C-$ | $-C_{12}H_{25}-n$ | CH(OH) | 1,4860 |
| 45 | $(CH_3)_3C-$ | $-C_6H_{21}-n$ | CH(OH) | 1,4837 |
| 46 | $(CH_3)_3C-$ | 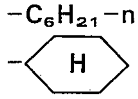 | CH(OH) | 1,5100 |

| Bsp. Nr. | $R^1$ | $R^2$ | A | Schmelzpunkt (°C) bzw. Brechungsindex $n_D^{20}$ |
|---|---|---|---|---|
| 47 | $(CH_3)_3C-$ | $-C_2H_5$ | CH(OH) | 1,5120 |
| 48 | $(CH_3)_3C-$ | $-C_{10}H_{21}-n$ | CH(OH) | 1,4800 |
| 49 | $(CH_3)_3C-$ | $-C_{18}H_{37}-n$ | CO | Öl |
| 50 | $(CH_3)_3C-$ | $-CH_2-CH(CH_3)_2$ | CH(OH) | 1,4925 |
| 51 | $(CH_3)_3C-$ | $-CH_2CH_2CH(CH_3)_2$ | CH(OH) | 1,4875 |
| 52 | $CH_3-C(CH_2F)_2-$ | $CH_3$ | CH(OH) | 25 |
| 53 | $CH_3-C(CH_2F)_2-$ | $-CH(CH_3)-C_2H_5$ | CH(OH) | Öl |
| 54 | $(CH_3)_3C-$ | $-CH_2-CH(CH_3)_2$ | CO | 1,4937 |
| 55 | $(CH_3)_3C-$ | $-CH_2CH_2CH(CH_3)_2$ | CO | 1,4893 |
| 56 | $CH_3-C(CH_2F)_2-$ | $-CH(CH_3)-C_2H_5$ | CO | Öl |
| 57 | $CH_3-C(CH_2F)_2-$ | $CH_3$ | CO | Öl |
| 58 | $CH_3-C(CH_2F)_2-$ | $-CH(CH_3)_2$ | CO | Öl |

*Anwendungsbeispiele*

In den nachfolgenden Beispielen werden die nachstehend angegebenen Verbindungen als Vergleichssubstanzen eingesetzt:

(A) 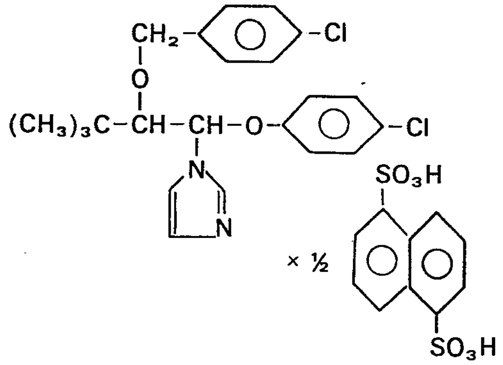

(B) 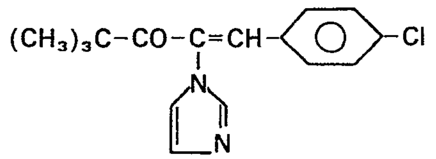

(C) 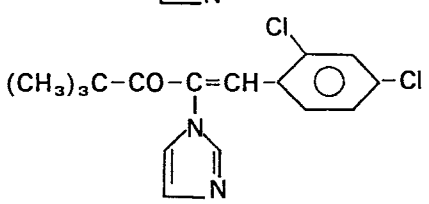

*Beispiel A*

Erysiphe-*Test (Gerste)/protektiv*

Lösungsmittel: 100 Gew.-Teile Dimethylformamid

Emulgator: 0,25 Gew.-Teile Alkylarylpolyglykoläther

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von *Erysiphe graminis f.sp.hordei* bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 d nach der Inokulation erfolgt die Auswertung.

Eine deutliche Überlegenheit in der Wirksamkeit gegenüber dem Stand der Technik zeigen bei diesem Test z.B. die Verbindungen gemäss folgender Herstellungsbeispiele: 6, 11, 12, 8, 1, 10, 13, 24, 27, 29, 33, 35, 39, 46, 36, 2, 38, 37 und 40.

Tabelle A
Erysiphe-*Test (Gerste) / protektiv*

| Wirkstoff | Wirkstoff-konzentration in der Spritzbrühe (Gew.-%) | Krankheitsbefall (%) der unbehandelten Kontrolle |
|---|---|---|
| (A) 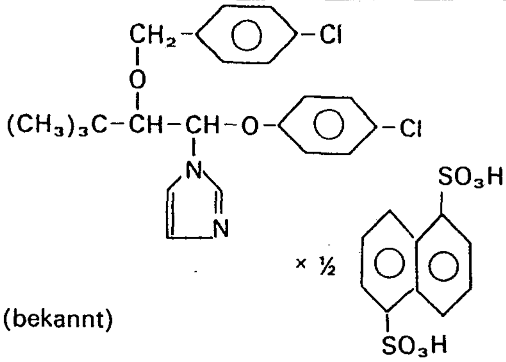 × ½ (bekannt) | 0,025 | 48,8 |
| 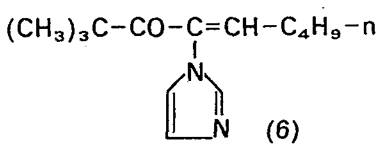 (6) | 0,025 | 0,0 |
| 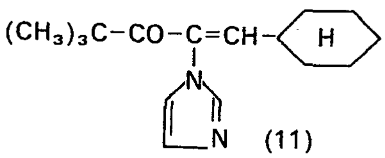 (11) | 0,025 | 8,8 |
| 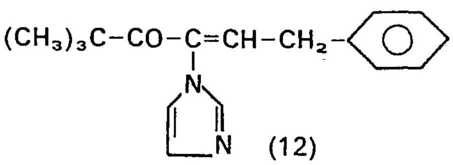 (12) | 0,025 | 0,0 |
| 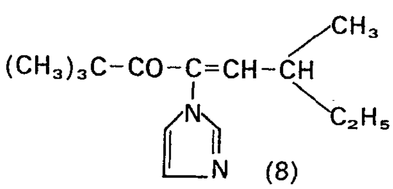 (8) | 0,025 | 12,5 |
| 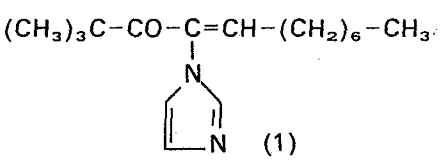 (1) | 0,025 | 12,5 |
| 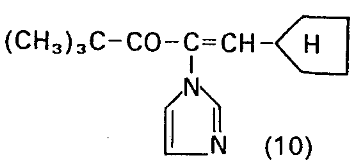 (10) | 0,025 | 0,0 |

Tabelle A *(Fortsetzung)*
Erysiphe-*Test (Gerste) / protektiv*

| Wirkstoff | Wirkstoff-konzentration in der Spritzbrühe (Gew.-%) | Krankheitsbefall (%) der unbehandelten Kontrolle |
|---|---|---|
| 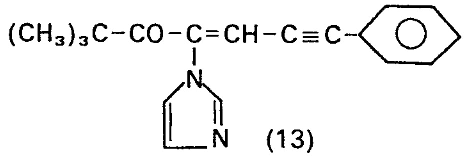 (13) | 0,025 | 0,0 |
| 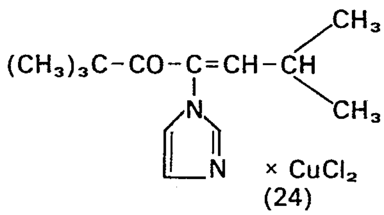 × $CuCl_2$ (24) | 0,025 | 0,0 |
| 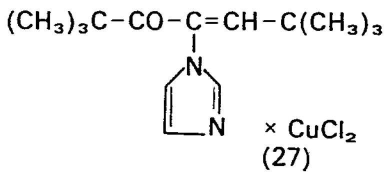 × $CuCl_2$ (27) | 0,025 | 16,3 |
| 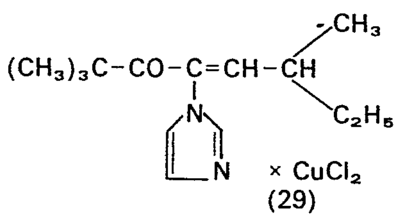 × $CuCl_2$ (29) | 0,025 | 0,0 |
| 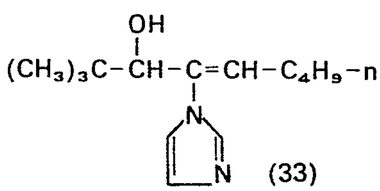 (33) | 0,025 | 0,0 |
| 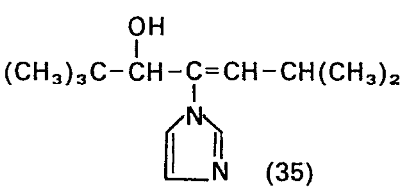 (35) | 0,025 | 16,3 |
| 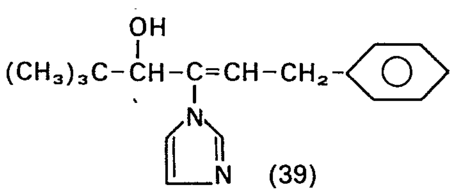 (39) | 0,025 | 0,0 |
| 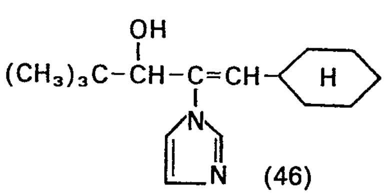 (46) | 0,025 | 0,0 |

Tabelle A *(Fortsetzung)*

Erysiphe-*Test (Gerste) / protektiv*

| Wirkstoff | Wirkstoff-konzentration in der Spritzbrühe (Gew.-%) | Krankheitsbefall (%) der unbehandelten Kontrolle |
|---|---|---|
| 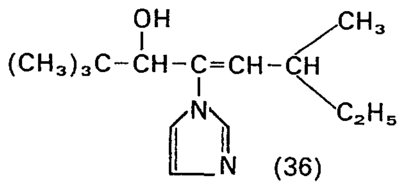 (36) | 0,025 | 0,0 |
| 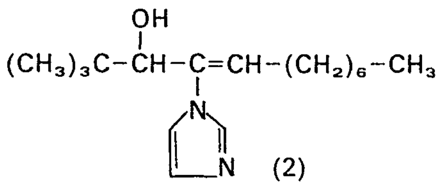 (2) | 0,025 | 0,0 |
| 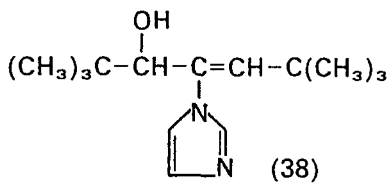 (38) | 0,025 | 0,0 |
| 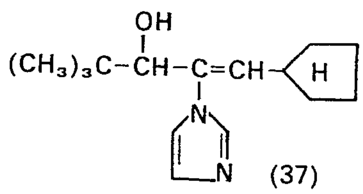 (37) | 0,025 | 0,0 |
| 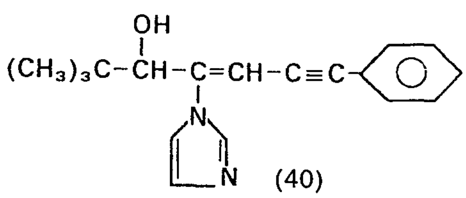 (40) | 0,025 | 0,0 |

*Beispiel B*

*Gerstenmehltau-Test* (Erysiphe graminis sp. hordei)/*systemisch (pilzliche Getreidesprosskrankheit)*

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 d nach der Aussaat, wenn die Gerstenpflanzen ihr erstes Blatt entfaltet haben, werden sie mit frischen Sporen von *Erysiphe graminis sp. hordei* bestäubt und bei 21-22° C und 80-90% rel. Luftfeuchte und 16stündiger Belichtung weiter kultiviert. Innerhalb von 6 d bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle.

Der Wirkstoff ist um so wirksamer je geringer der Mehltaubefall ist.

Eine deutliche Überlegenheit in der Wirksamkeit gegenüber dem Stand der Technik zeigen bei diesem Test z.B. die Verbindungen gemäss folgender Herstellungsbeispiele: 46.

Tabelle B

*Gerstenmehltau-Test* (Erysiphe graminis sp. hordei) / *systemisch*

| Wirkstoff | Wirkstoff-konzentration im Beizmittel (Gew.-%) | Beizmittel-aufwandmenge Saatgut (g/kg) | Befall (%) der unbehandelten Kontrolle |
|---|---|---|---|
| (A) 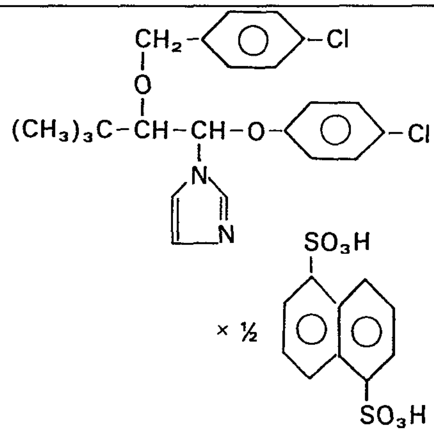 × ½ | 25 | 10 | 100 |
| 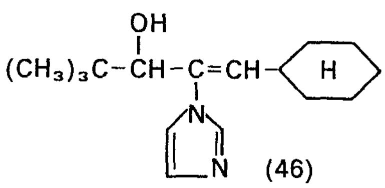 (46) | 25 | 4 | 12,5 |

*Beispiel C*

Erysiphe-*Test (Gurken)/protektiv*

Lösungsmittel: 4,7 Gew.-Teile Aceton

Emulgator: 0,3 Gew.-Teile Alkylarylpolyglykoläther

Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 h im Gewächshaus. Dann werden sie zur Inokulation mit Konidien des Pilzes *Erysiphe cichoracearum* bestäubt. Die Pflanzen werden anschliessend bei 23 bis 24° C und bei einer relativen Luftfeuchtigkeit von ca. 75% im Gewächshaus aufgestellt.

Nach 12 d wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber dem Stand der Technik zeigen bei diesem Test z.B. die Verbindungen gemäss folgender Herstellungsbeispiele: 33 und 46.

Tabelle C

Erysiphe-*Test (Gurken) / protektiv*

| Wirkstoff | Befall (%) bei einer Wirkstoffkonzentration von 0,0005% |
|---|---|
| (B) 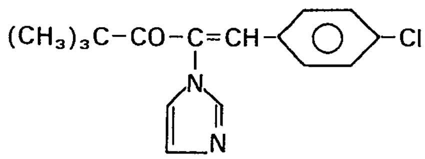 | 84 |
| (C) 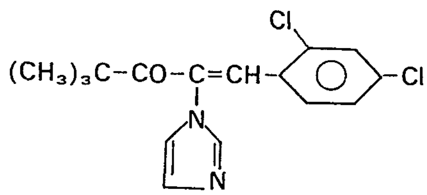 | 100 |
| 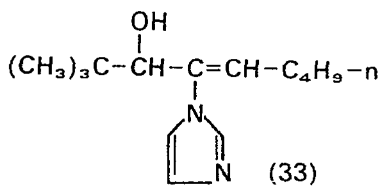 (33) | 37 |
| 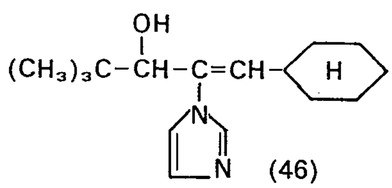 (46) | 37 |

*Beispiel D*

Phytophthora-*Test (Tomaten)/protektiv*

Lösungsmittel: 4,7 Gew.-Teile Aceton

Emulgator: 0,3 Gew.-Teile Alkylarylpolyglykoläther

Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Tomatenpflanzen mit 2 bis 4 Laubblättern bis zur Tropfnässe. Die Pflanzen verbleiben 24 h bei 20° C und einer relativen Luftfeuchtigkeit von 70% im Gewächshaus. Anschliessend werden die Tomatenpflanzen mit einer wässerigen Sporensuspension von *Phytophthora infestans* inokuliert. Die Pflanzen werden in eine Feuchtkammer mit einer 100%igen Luftfeuchtigkeit und einer Temperatur von 18 bis 20° C gebracht.

Nach 5 d wird der Befall der Tomatenpflanzen bestimmt. Die erhaltenen Boniturwerte werden auf Prozent Befall umgerechnet. 0% bedeutet keinen Befall, 100% bedeutet, dass die Pflanzen vollständig befallen sind.

Eine deutliche Überlegenheit in der Wirksamkeit gegenüber dem Stand der Technik zeigen bei diesem Test z.B. die Verbindungen gemäss folgender Herstellungsbeispiele: 26, 27, 28 und 29.

Tabelle D
Phytophthora-*Test (Tomaten) / protektiv*

| Wirkstoff | Befall (%) bei einer Wirkstoffkonzentration von 0,005% |
|---|---|
| 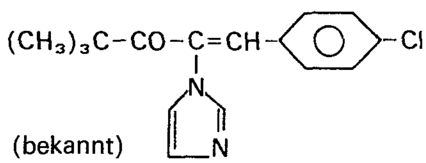 (B) (bekannt) | 21 |
| 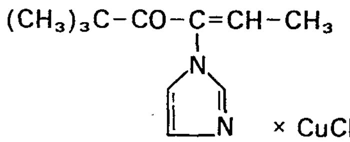 × $CuCl_2$ (26) | 2 |
| 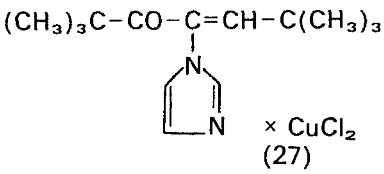 × $CuCl_2$ (27) | 4 |
| 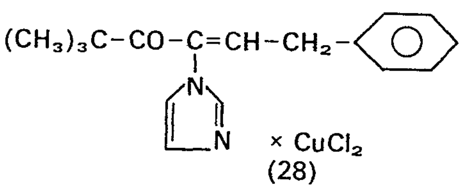 × $CuCl_2$ (28) | 0 |
| 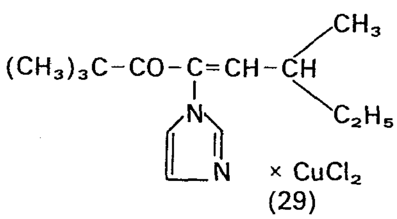 × $CuCl_2$ (29) | 2 |

**Patentansprüche**

1. Imidazolylvinylketone und -carbinole der allgemeinen Formel

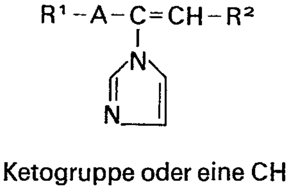 (I)

in welcher

A für die Ketogruppe oder eine CH(OH)-Gruppierung steht,

$R^1$ für tert.-Butyl, Chlor-tert.-butyl, Fluor-tert.-butyl, Dichlor-tert.-butyl und Difluor-tert.-butyl steht, und

$R^2$ für Alkyl mit 1 bis 29 Kohlenstoffatomen, für Cycloalkyl und Cycloalkenyl mit 5 bis 7 Kohlenstoffatomen und für Halogenalkyl mit 1 bis 18 Kohlenstoff- und 1 bis 5 Halogenatomen steht, weiterhin für Alkoxyalkyl und Alkylmercaptoalkyl mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatommen im Alkylteil steht, sodann für Hydroxyalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen und durch Alkyl, Alkoxy und Alkylthio mit jeweils 1 bis 4 Kohlenstoffatomen, und ausserdem noch für Benzyl steht, welches im Phenylteil durch die weiter oben genannten Phenylsubstituenten und die 1,1,2-Trifluor-2-chlorethoxy-Gruppe und im Alkylteil durch Cyano substituiert sein kann, und schliesslich noch für die Indenyl-, die Fluorenyl-, die Diphenylmethyl- und die Triphenylmethylgruppe steht,
und deren physiologisch verträglichen Säureadditionssalze sowie Metallsalzkomplexe.

2. Verfahren zur Herstellung von Imidazolylvinylketonen und -carbinolen der allgemeinen Formel

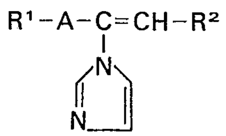 (I)

in welcher

A für die Ketogruppe oder eine CH(OH)-Gruppierung steht,

$R^1$ für tert.-Butyl, Chlor-tert.-butyl, Fluor-tert.-butyl, Dichlor-tert.-butyl und Difluor-tert.-butyl steht, und

$R^2$ für Alkyl mit 1 bis 29 Kohlenstoffatomen, für Cycloalkyl und Cycloalkenyl mit 5 bis 7 Kohlenstoffatomen und für Halogenalkyl mit 1 bis 18 Kohlenstoff- und 1 bis 5 Halogenatomen steht, weiterhin für Alkoxyalkyl und Alkylmercaptoalkyl mit jeweils 1 bis 4 Kohlenstoffatomen in jedem Alkylteil steht, für Dialkylaminoalkyl mit 1 bis 4 Kohlenstoffatomen in den Alkylresten der Aminogruppe und 1 bis 18 Kohlenstoffatomen im Alkylteil steht, sodann für Hydroxyalkyl mit 1 bis 18 Kohlenstoffatomen steht, für Alkenyl, Alkinyl und Alkeninyl mit jeweils bis zu 6 Kohlenstoffatomen steht, wobei die drei letztgenannten Reste substituiert sein können durch Hydroxy, Alkoxy mit 1 bis 4 Kohlenstoffatomen und durch Phenyl, wobei letzteres selbst noch substituiert sein kann durch Halogen und durch Alkyl, Alkoxy und Alkylthio mit jeweils 1 bis 4 Kohlenstoffatomen, und ausserdem noch für Benzyl steht, welches im Phenylteil durch die weiter oben genannten Phenylsubstituenten und die 1,1,2-Trifluor-2-chlorethoxygruppe und im Alkylteil durch Cyano substituiert sein kann, und schliesslich noch für die Indenyl-, die Fluorenyl-, die Diphenylmethyl- und die Triphenylmethylgruppe steht,
dadurch gekennzeichnet, dass man Ketoenamine der Formel

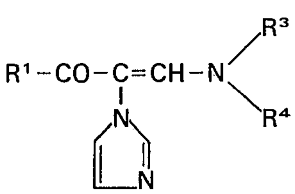 (II)

in welcher

$R^1$ die oben angegebene Bedeutung hat, und

$R^3$ und $R^4$ gleich oder verschieden sind und für Alkyl mit 1 bis 4 Kohlenstoffatomen stehen,
mit magnesium-organischen Verbindungen der Formel

$$Hal-Mg-R^2 \qquad (III)$$

in welcher

$R^2$ die oben angegebene Bedeutung hat, und

Hal für Halogen steht,

in Gegenwart eines Lösungsmittels umsetzt, und gegebenenfalls die entstehenden Ketoderivate der Formel (I) in üblicher Weise reduziert, und weiterhin noch an die so erhaltenen Verbindungen gegebenenfalls anschliessend eine Säure oder ein Metallsalz addiert.

3. Fungizide Mittel, gekennzeichnet durch einen Gehalt an mindestens einem Imidazolylvinylketon oder -carbinol der Formel (I) in Ansprüchen 1 und 2.

4. Verfahren zur Bekämpfung von Pilzen, dadurch gekennzeichnet, dass man Imidazolylvinylketone oder -carbinole der Formel (I) in Ansprüchen 1 und 2 auf Pilze oder ihren Lebensraum einwirken lässt.

5. Verwendung von Imidazolylvinylketonen oder -carbinolen der Formel (I) in Ansprüchen 1 und 2 zur Bekämpfung von Pilzen.

6. Verfahren zur Herstellung von fungiziden Mitteln, dadurch gekennzeichnet, dass man Imidazolylvinylketone oder -carbinole der Formel (I) in Ansprüchen 1 und 2 mit Streckmitteln und/oder oberflächenaktiven Mitteln vermischt.

## Claims

1. Imidazolylvinyl ketones and carbinols of the general formula

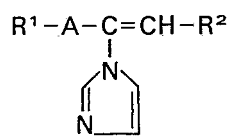 (I)

in which

A represents the keto group or a CH(OH) grouping,

$R^1$ represents tert.-butyl, chloro-tert.-butyl, fluoro-tert.-butyl, dichloro-tert.-butyl and difluoro-tert.-butyl, and

$R^2$ represents alkyl with 1 to 29 carbon atoms, cycloalkyl and cycloalkenyl with 5 to 7 carbon atoms and halogenoalkyl with 1 to 18 carbon and 1 to 5 halogen atoms, furthermore represents alkoxyalkyl and alkylmercaptoalkyl with in each case 1 to 4 carbon atoms in each alkyl part, and represents dialkylaminoalkyl with 1 to 4 carbon atoms in the alkyl radicals on the amino groups and 1 to 18 carbon atoms in the alkyl part, also represents hydroxyalkyl with 1 to 18 carbon atoms, and represents alkenyl, alkinyl and alkeninyl with in each case up to 6 carbon atoms, it being possible for the three last-mentioned radicals to be substituted by hydroxyl, alkoxy with 1 to 4 carbon atoms and by phenyl, it being

possible for the latter to itself also be substituted by halogen and by alkyl, alkoxy and alkylthio with in each case 1 to 4 carbon atoms, and in addition also represents benzyl which can be substituted in the phenyl part by the phenyl substituents mentioned further above and the 1,1,2-trifluoro-2-chloroethoxy group and in the alkyl part by cyano, and finally also represents the indenyl, the fluorenyl, the diphenylmethyl and the triphenylmethyl group, and physiologically acceptable acid addition salts and metal salt complexes thereof.

2. Process for the preparation of imidazolylvinyl ketones and carbinols of the general formula

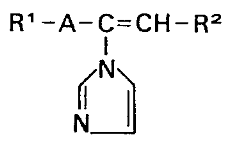 (I)

in which

A represents the keto group or a CH(OH) grouping,

$R^1$ represents tert.-butyl, chloro-tert.-butyl, fluoro-tert.-butyl, dichloro-tert.-butyl and difluoro-tert.-butyl, and

$R^2$ represents alkyl with 1 to 29 carbon atoms, cycloalkyl and cycloalkenyl with 5 to 7 carbon atoms and halogenoalkyl with 1 to 18 carbon and 1 to 5 halogen atoms, furthermore represents alkoxyalkyl and alkylmercaptoalkyl with in each case 1 to 4 carbon atoms in each alkyl part, and represents dialkylaminoalkyl with 1 to 4 carbon atoms in the alkyl radicals on the amino groups and 1 to 18 carbon atoms in the alkyl part, also represents hydroxyalkyl with 1 to 18 carbon atoms, and represents alkenyl, alkinyl and alkeninyl with in each case up to 6 carbon atoms, it being possible for the three last-mentioned radicals to be substituted by hydroxyl, alkoxy with 1 to 4 carbon atoms and by phenyl, it being possible for the latter to itself also be substituted by halogen and by alkyl, alkoxy and alkylthio with in each case 1 to 4 carbon atoms, and in addition also represents benzyl which can be substituted in the phenyl part by the phenyl substituents mentioned further above and the 1,1,2-trifluoro-2-chloroethoxy group and in the alkyl part by cyano, and finally also represents the indenyl, the fluorenyl, the diphenylmethyl and the triphenylmethyl group, characterised in that keto-enamines of the formula

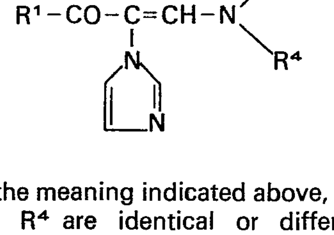 (II)

in which

$R^1$ has the meaning indicated above, and

$R^3$ and $R^4$ are identical or different and represent alkyl with 1 to 4 carbon atoms,

are reacted with organo-magnesium compounds of the formula

$$Hal-Mg-R^2 \quad (III)$$

in which

$R^2$ has the meaning indicated above, and

Hal represents halogen,

in the presence of a solvent, and, if appropriate, the keto derivatives of the formula (I) formed are reduced in the customary manner, and, furthermore, an acid or a metal salt is also optionally subsequently added onto the compounds thus obtained.

3. Fungicidal agents, characterised in that they contain at least one imidazolylvinyl ketone or carbinol of the formula (I) in claims 1 and 2.

4. Process for combating fungi, characterised in that imidazolylvinyl ketones or carbinols of the formula (I) in claims 1 and 2 are allowed to act on fungi or their environment.

5. Use of imidazolylvinyl ketones or carbinols of the formula (I) in claims 1 and 2 for combating fungi.

6. Process for the preparation of fungicidal agents, characterised in that imidazolylvinyl ketones or carbinols of the formula (I) in claims 1 and 2 are mixed with extenders and/or surface-active agents.

## Revendications

1. Imidazolylvinylcétones et -carbinols de formule générale

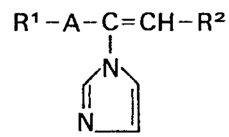 (I)

dans laquelle

A représente le groupe céto ou un groupement CH(OH),

$R^1$ représente un groupe tert.-butyle, chloro-tert.-butyle, fluoro-tert.-butyle, dichloro-tert.-butyle et difluoro-tert.-butyle, et

$R^2$ représente un groupe alkyle en $C_1$-$C_{29}$, cycloalkyle et cycloalcényle contenant de 5 à 7 atomes de carbone et halogénoalkyle contenant de 1 à 18 atomes de carbone et de 1 à 5 atomes d'halogènes, ou encore alcoxyalkyle et alkylmercaptoalkyle contenant chacun 1 à 4 atomes de carbone dans chaque partie alkyle, dialkylaminoalkyle contenant 1 à 4 atomes de carbone dans les restes alkyle du groupe amino et 1 à 18 atomes de carbone dans la partie alkyle, ou encore hydroxyalkyle en $C_1$-$C_{18}$, alcényle, alcynyle et alcénynyle contenant chacun jusqu'à 6 atomes de carbone, les trois restes mentionnés en dernier pouvant être substitués par des groupes hydroxy, alcoxy en $C_1$-$C_4$ et phényle, ce dernier pouvant lui-même encore être substitué par des halogènes et par des groupes alkyle, alcoxy et alkylthio contenant chacun 1 à 4 atomes de carbone et, en outre, par un groupe benzyle qui peut être substitué dans la partie phényle par les substituants du groupe phényle mentionnés ci-dessus et par le groupe 1,1,2-trifluoro-2-chloréthoxy, et dans la partie alkyle par un groupe cyano et, finalement, encore un groupe

indényle, fluorényle, diphénylméthyle et triphénylméthyle,
et leurs sels formés par addition avec des acides tolérés par l'organisme et complexes de sels métalliques.

2. Procédé de préparation des imidazolylvinylcétones et -carbinols de formule générale

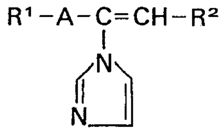 (I)

dans laquelle

A représente le groupe céto ou un groupement CH(OH),

$R^1$ représente un groupe tert.-butyle, chloro-tert.-butyle, fluoro-tert.-butyle, dichloro-tert.-butyle et difluoro-tert.-butyle, et

$R^2$ représente un groupe alkyle contenant de 1 à 29 atomes de carbone, cycloalkyle et cycloalcényle contenant de 5 à 7 atomes de carbone et halogénoalkyle contenant 1 à 18 atomes de carbone et 1 à 5 atomes d'halogènes, ou encore alcoxyalkyle et alkylmercaptoalkyle contenant chacun 1 à 4 atomes de carbone dans chaque partie alkyle, dialkylaminoalkyle contenant 1 à 4 atomes de carbone dans les restes alkyle du groupe amino et 1 à 18 atomes de carbone dans la partie alkyle, ou encore hydroxyalkyle contenant 1 à 18 atomes de carbone, alcényle, alcynyle et alcénynyle contenant chacun jusqu'à 6 atomes de carbone, les trois restes mentionnés en dernier pouvant être substitués par des groupes hydroxy, alcoxy en $C_1$-$C_4$ et phényle, ce dernier pouvant lui-même encore être substitué par des halogènes et par des groupes alkyle, alcoxy et alkylthio contenant chacun 1 à 4 atomes de carbone, et, de plus, encore benzyle qui peut être substitué dans la partie phényle par les substituants du groupe phényle mentionnés ci-dessus et par le groupe 1,1,2-trifluoro-2-chloréthoxy, et dans la partie alkyle par un groupe cyano et, finalement, encore le groupe indényle, le groupe fluorényle, le groupe diphénylméthyle et le groupe triphénylméthyle, caractérisé en ce que l'on fait réagir des cétoénamines de formule

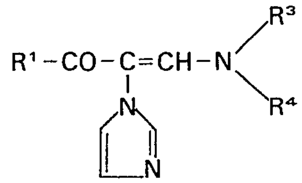 (II)

dans laquelle

$R^1$ a les significations indiquées ci-dessus, et

$R^3$ et $R^4$, identiques ou différents, représentent des groupes alkyle en $C_1$-$C_4$, avec des composés organomagnésiens de formule

$$Hal-Mg-R^2 \qquad (III)$$

dans laquelle

$R^2$ a la signification indiquée ci-dessus, et Hal représente un halogène,
en présence d'un solvant et, le cas échéant, on réduit de la manière habituelle les dérivés cétoniques de formule (I) ainsi obtenus et, en outre, le cas échéant, sur les composés ainsi obtenus, on fixe par addition un acide ou un sel métallique.

3. Produits fongidices caractérisés en ce qu'ils contiennent au moins une imidazolylvinylcétone ou un imidazolylvinylcarbinol de formule (I) dans les revendications 1 et 2.

4. Procédé pour combattre les mycètes, caractérisé en ce que l'on fait agir sur les mycètes ou leurs habitats des imidazolylvinylcétones ou -carbinols de formule (I) dans les revendications 1 et 2.

5. Utilisation des imidazolylvinylcétones ou -carbinols de formule (I) dans les revendications 1 et 2 pour combattre les mycètes.

6. Procédé de préparation de produits fongicides, caractérisé en ce que l'on mélange les imidazolylvinylcétones ou -carbinols de formule (I) dans les revendications 1 et 2 avec des diluants et/ou des agents tensio-actifs.